Europäisches Patentamt

European Patent Office

Office européen des brevets

(11) Veröffentlichungsnummer: **0 263 361**
**A2**

(12) **EUROPÄISCHE PATENTANMELDUNG**

(21) Anmeldenummer: 87113939.0

(22) Anmeldetag: 24.09.87

(51) Int. Cl.4: **A61K 39/29**

Patentansprüche für folgenden Vertragsstaaten:
GR + ES.

(30) Priorität: 02.10.86 DE 3633550

(43) Veröffentlichungstag der Anmeldung:
**13.04.88 Patentblatt 88/15**

(84) Benannte Vertragsstaaten:
**AT BE CH DE ES FR GB GR IT LI LU NL SE**

(71) Anmelder: **BEHRINGWERKE
AKTIENGESELLSCHAFT
Postfach 1140
D-3550 Marburg/Lahn(DE)**

(72) Erfinder: **Gregersen, Jens-Peter, Dr.
Am Nebelrock 6
D-3551 Lahntal(DE)**
Erfinder: **Noll, Gabriele, Dr.
Barfüsserstrasse 46/47
D-3550 Marburg(DE)**
Erfinder: **Mauler, Rudolf, Dr.
Finkenstrasse 6
D-3550 Marburg(DE)**

(74) Vertreter: **Becker, Heinrich Karl Engelbert, Dr.
et al
HOECHST AKTIENGESELLSCHAFT Central
Patent Department P.O. Box 80 03 20
D-6230 Frankfurt am Main 80(DE)**

(54) **Vaccine gegen Hepatitis A.**

(57) Es wird ein Verfahren zur Herstellung einer Vaccine gegen Hepatitis A beschrieben, worin ein aus menschlichem Material gewonnenes Hepatitis A-Virus fünfzehn- bis dreißigmal auf menschlichen Zellen passagiert wird.

Eine Vaccine, die ein auf diese Weise kultiviertes Virus enthält, ist vorzugsweise zur oralen Administration geeignet.

EP 0 263 361 A2

## Vaccine gegen Hepatitis A

Die Erfindung betrifft eine Vaccine gegen Hepatitis A, welche Hepatitis A-Virus enthält, das aus menschlichem Material isoliert und ausschließlich auf Zellen menschlichen Ursprungs vermehrt wurde. Diese Vaccine ist auch zur oralen Applikation geeignet.

Hepatitis A ist eine weltweit verbreitete Infektionskrankheit des Menschen, die insbesondere in Ländern mit geringem hygienischen Standard endemisch ist. Der Erreger (Hepatitis A-Virus, HAV) ist ein Picornavirus aus der Subfamilie der Enteroviren. Die Übertragung der Infektion geschieht in der Regel durch fäkal-orale Schmierinfektion mit dem Trinkwasser oder durch kontaminierte Lebensmittel. Während Kinder nach Infektion häufig symptomlos bleiben oder milde erkranken, erleiden 50 % der infizierten Erwachsenen eine akute Hepatitis. In entwickelten Ländern sind etwa 20-25 % der Hepatitis-Erkrankungen vom A-Typ, wobei besonders junge Erwachsene in Wohnheimen, Lagern und militärischen Einrichtungen sowie Reisende in Entwicklungsländer betroffen sind.

In EP-A-0 008 559 ist ein Verfahren zur Züchtung eines menschlichen Hepatitis A-Virus in Zellkultur nach vorheriger Viruspassage in subhumanen Primaten beschrieben, um solcherart vermehrtes Virus nach Abtötung oder Abschwächung seiner Virulenz als Vaccine zu verwenden.

In EP-A-0 025 745 ist ein Verfahren der direkten in-vitro Vermehrung eines Hepatitis A-Virus ohne vorherige Primaten-Passage beschrieben.

Eine Modifikation letztgenannter Methode unter anschließender Verwendung von Zellen menschlichen Ursprungs wird auch im US-Patent 4,506,016 (entspricht EP-A-0 074 119) beschrieben. Die Entwicklung einer abgeschwächten Lebendvirusvaccine ist im Detail in Proc. Soc. Exp. Biol. Med. 170, 8-14 (1982) und Proc. Soc. Exp. Biol. Med. 172, 357-363 (1983) erläutert: Nach Zellkulturpassagen attenuiertes Virus wurde auf intravenösem Wege an Affen verabreicht, die daraufhin Antikörper gegen Hepatitis A entwickelten, welche vor eine Erkrankung durch nicht abgeschwächtes Virus schützten.

Ein Impfstoff der letztgenannten Art hat jedoch den Nachteil, daß er auf parenteralem Wege verabreicht werden muß.

Wünschenswert wäre eine Vaccine, die nach oraler Gabe zur Ausbildung einer Immunität führt. Ein solcher Impfstoff wäre nicht nur einfacher zu applizieren, sondern könnte auch eine der natürlichen Immunität entsprechende Immunantwort stimulieren, die durch enterale Immunmechanismen das weitere Eindringen und die Vermehrung von virulentem Hepatitis A-Virus im Körper verhindert.

Die veröffentlichte internationale Patentanmeldung WO 86/01826 (Appl. No. PCT. US 85/01769) beschreibt die Isolierung des Hepatitis A-Virusstammes HM 175 und dessen Verwendung als Lebendvaccine, wobei auch eine orale Anwendung beschrieben wird. Dieser Virusstamm weist jedoch den Nachteil auf, daß das verwendete Virus in primären Nierenzellen von Afrikanischen Grünen Meerkatzen gezüchtet wird und damit die Möglichkeit besteht, daß andere unerwünschte Viren aus Affenzellen in der daraus gewonnenen Vaccine enthalten sein können. Insbesondere besteht eine Gefährdung durch Viren der Retrovirus-Familie, die eine nahe Verwandtschaft zum AIDS-Erreger aufweisen. Die Anwesenheit solcher Viren in einer Lebendvaccine würde deren Anwendung beim Menschen ausschließen.

Aufgabe der Erfindung war es, eine oral anwendbare Lebendvaccine zu entwickeln, die vor einer Erkrankung durch Hepatitis A-Virus schützt und aufgrund der Verwendung von geprüften, von vermehrbaren Fremdviren freien Zellkulturen menschlichen Ursprungs nicht mit dem Risiko behaftet ist, unerwünschte, vermehrbare Viren zu enthalten.

Überraschenderweise wurde gefunden, daß ein aus menschlichem Material gewonnenes Hepatitis A-Virus nach 15 bis 30 Passagen auf menschlichen Zellen als Antigen in einem auch oral anwendbaren Impfstoff geeignet ist.

Gegenstand der Erfindung ist daher eine Vaccine gegen Hepatitis A enthaltend eine wirksame Menge eines Hepatitis A-Virus, das aus menschlichem Material isoliert und ausschließlich in Zellen menschlichen Ursprungs vermehrt wurde, dadurch gekennzeichnet, daß zur Vermehrung des Virus fünfzehn-bis dreißigmal passagiert wurde.

Vorzugsweise wird zwanzig-bis dreißigmal passagiert. Eine solche Vaccine kann vorteilhaft oral angewandt werden.

Als Ausgangsmaterial wird ein Hepatitis A-Virus benutzt, welches aus menschlichem virushaltigen Material stammte. Eine solches virushaltiges Material kann beispielsweise Kot, Blut, Speichel, Urin oder ein Gewebeextrakt sein. Darin enthaltenes Virus oder Teile des Virus werden direkt in ein von menschlichem Zellmaterial ausgehendes in-vitro Vermehrungssystem verbracht, mit dem Ziel, Virus und/oder immunologisch relevante Virusanteile zu vermehren. Als Vermehrungssystem eignen sich eine Vielzahl von primären, kontinuierlich kultivierten oder permanenten Zellkulturen. Vorzugsweise werden jedoch Zellkulturen aus Zellen menschlichen Ursprungs verwendet, die bereits für die Herstellung anderer beim Menschen angewandter Virusvaccinen verwendet wurden und von anderen infektiösen Agentien frei sind.

Während dieser in-vitro Vermehrung wird die pathogene Wirkung der Hepatitis A-Viren abgeschwächt. Nach ausreichender Adaptierung der Viren an ein solches Vermehrungssystem können nun Hepatitis A-Viren oder ihre immunologisch relevanten Anteile in ausreichender Menge erhalten werden, um damit nach oraler Verabreichung eine - schützende Immunantwort in vivo auszulösen, ohne eine krankmachende Wirkung zu erhalten.

Überraschenderweise erwies sich die orale Verabreichung dabei als vorteilhafter als eine parenterale Gabe. Obwohl der natürliche Infektionsweg nachgeahmt wurde, verursachte eine erfindungsgemäße Vaccine nach oraler Gabe im Tierexperiment keine Hepatitis, während nach parenteraler Gabe (i.v.) derselben Viruspräparation Anzeichen einer leichten Hepatitis festzustellen war. Somit kann also die orale Verabreichung dieser Hepatitis A-Lebendvaccine als sicherer angesehen werden als eine parenterale.

Ein portionierter, ausreichend virushaltiger, sterilfiltrierter Kulturüberstand stellt bereits eine geeignete Zubereitung dar, die den geforderten Zweck erzielt. Jedoch bietet es sich aus praktischen Gründen an, das enthaltene Virus durch Zusatz von virusstabilisierenden Substanzen oder/und durch Gefriertrocknung haltbarer zu machen, um es nach Rekonstituierung direkt einzuflößen oder in einem pharmakologisch unbedenklichen Träger (beispielsweise einem Zuckerwürfel) zu verabreichen.

Im Folgenden wird beispielhaft dargestellt, wie eine erfindungsgemäße, vorzugsweise oral anwendbare Vaccine. die vor der Erkrankung durch Hepatitis A-Virus schützt, hergestellt werden kann. Weiterhin wird ihre Anwendung und Wirksamkeit im Versuchstier erläutert.

## Beispiel 1

Virusvermehrung in vitro

Ein wie im USP 4,506,016 beschrieben gewonnenes Hepatitis A-Virusisolat (HAV/GBM), wurde durch wiederholte, aufeinanderfolgende in vitro-Passagen vermehrt.

Dies geschah durch die Inokulation virushaltiger Kulturüberstände in Kulturen aus menschlichen embryonalen Nieren-oder Lungenfibroblasten, die entsprechend den üblichen Labortechniken in Eagles Medium unter Zusatz von 10 % foetalem Kälberserum gehalten wurden. Nach etwa einwöchiger Inkubation der Kulturen bei 37°C waren große Mengen ausgeschleuster Viruspartikel im Kulturüberstand und Virusantigene in der Zellkultur nachweisbar.

## Beispiel 2

Adaptation von Hepatitis A-Virus an Zellkulturen

Durch wiederholte Überimpfung von virushaltigen Kulturüberständen aus einer Zellkultur in neue, nicht infizierte Zellen wird eine zunehmende Steigerung der Virusausbeuten im Kulturüberstand erreicht. Während anfänglich kaum nachweisbare Mengen an infektiösem Virus erhalten werden, steigern sich diese Ausbeuten mit zunehmender Zahl der Passagen. Außerdem erscheint infektiöses Virus zunehmend früher im Kulturüberstand, so daß die Vermehrungszeit auf wenige Tage verkürzt werden kann. In ähnlicher Weise ist Hepatitis A-Virus an eine Zellkultur adaptierbar, indem eine infizierte Zellkultur kontinuierlich weitervermehrt wird. Die Abstände zwischen den einzelnen Zellpassagen können dabei von anfänglich mehreren Wochen auf wenige Tage verkürzt werden, da im Zuge fortschreitender Adaptierung das Virus zunehmend - schneller vermehrt wird. Beide Methoden sind geeignet, innerhalb von etwa 20 Viruspassagen (beziehungsweise Zellpassagen) Virusausbeuten von $10^6$ bis $10^5$ infektiösen Partikeln pro Milliliter Kulturüberstand zu erhalten.

## Beispiel 3

Herstellung serumfreier Viruspräparationen

Nach Infektion annähernd konfluent ausgewachsener MRC-5-Zellen mit Hepatitis A-Virus durch Wechsel des Kulturmediums durch ein gleichwertiges Medium mit 5 % foetalem Kälberserum und darin enthaltenem Hepatitis A-Virus ($10^3$ -$10^7$ $KID_{50}$ml) wurden diese Zellkulturen einige Tage (3 - 6 Tage) bei 37°C inkubiert. Danach wurde das Kulturmedium entfernt und die Zellen mit serumfreien Medium dreimal gewaschen und mit serumfreien Medium erneut versorgt. Nach weiteren 3 bis 7 Tagen wurde dieser Kulturüberstand geerntet und dessen Virusgehalt bestimmt.

## Beispiel 4

Immunoperoxidase-Test zum Nachweis und zur Mengenbestimmung infektiöser Hepatitis A-Viren

Von infizierten Zellkulturen wurde 3 Wochen nach Infektion der Kulturüberstand entfernt und eine 4 %ige Formalinlösung in PBS (phosphatgepuffertes Kochsalzlösung, pH 7,2) auf die verbleibenden Zellen gegeben. Nach mindestens 12-stündiger Einwirkung bei 4°C wurde die Formalinlösung entfernt und durch eine 1 %ige ®Triton X 100-Detergenslösung in PBS ersetzt. Nachdem diese 30 Minuten bei Raumtemperatur einwirken konnte, wurde sie entfernt und mit PBS unter Zusatz von 1 % foetalem Kälberserum (Waschpuffer) verbleibende Detergensreste weggewaschen. Auf die nun fixierten und aufgeschlossenen Zellen wurde eine gereinigte IgG-Präparation mit einem hohen Anteil von Antikörpern gegen Hepatitis A-Virus, markiert mit Peroxidase gegeben, die in Waschpuffer verdünnt wurde. Nach einer Stunde Inkubation mit Aminoethylcarbazol (0,4 mg/ml) in einem Acetatpuffer pH 5,5 mit 0,003 % $H_2O_2$ zeigten infizierte Zellen eine rosa bis braunrote Farbe, die im Mikroskop beobachtet werden konnte, während nicht infizierte Zellen ungefärbt blieben. Zur Bestimmung des Gehaltes an infektiösen Viruspartikeln in einer Viruspräparation wird diese in 10er-Stufen verdünnt und mit jeder Verdünnungsstufe von $10^{-1}$ bis $10^{-7}$ werden 8 Zellkulturen in einer Mikrotestplatte inokuliert. Nach 3-wöchiger Inkubation bei 37°C wird der Infektionsnachweis wie oben beschrieben durchgeführt, um aus der Anzahl infizierter Kulturen mit bekannten statistischen Methoden die Menge infektiöser Einheiten, angegeben als kultur-infektiöse Dosis 50 % ($KID_{50}$), zu berechnen.

Beispiel 5

Schutzversuche in Schimpansen

Zur Überprüfung der Eignung als Vaccine wurde virushaltiger Kulturüberstand der 2., 20., 31. und 51. Kulturpassage Schimpansen inokuliert. In wöchentlichen Abständen wurden dann Serumproben entnommen, um diese auf Antikörper gegen Hepatitis A und die darin befindlichen Leberenzymspiegel zu testen. In Abständen von 1 - 3 Wochen wurden Leberbiopsien entnommen, um histologische Anzeichen einer Hepatitis zu entdecken. Die Verabreichung von $10^{5,0}$ $KID_{50}$ Hepatitis A-Virus der 2. Kulturpassage führte zu einem Anstieg der Leberenzyme, insbesondere der GGTP (gamma-Glutamyltranspeptidase)-Werte und zu Anzeichen einer akuten Hepatitis in der Biopsie. Die Gabe der 20. Kulturpassage ($10^{7,8}$ $KID_{50}$ Hepatitis A-Virus, oral verabreicht) hingegen führte zu einer Bildung von Antikörpern gegen Hepatitis A ohne Anstieg der Leberenzyme und zu keinem Anzeichen einer Hepatitis in der histologischen Beurteilung der entnommenen Leberbiopsien. Eine gleiche Menge derselben Viruspräparation wurde ebenfalls parenteral einem Schimpansen verabreicht ($10^{7,8}$ $KID_{50}$, 20. Kulturpassage, intravenös). Im Gegensatz zu einem oral geimpften Tier entwickelte dieser Affe noch eine leichte, akute Hepatitis und verdeutlichte damit den Vorteil der oralen Vaccination. Mit Virus der 31. und 51. Kulturpassage wurde keine Antikörperbildung hervorgerufen. In einer Belastungsinfektion mit Virus der 2. Kulturpassage, welches sich als pathogen erwiesen hatte, konnte der Nachweis geführt werden, daß ein Schimpanse nach vorheriger Gabe der 20. Kulturpassage gegen eine Hepatitis A geschützt war (Tabelle 1).

TABELLE 1

| Schimpanse | Kulturpassage | HAV-Inokulum Dosis $(KID_{50})$ | Inokulation | GGTP (U/l) Normalwerte | max. Werte | Anti-HAV-Antikörper HAVAB (IU/ml) maximal | Histopathologie |
|---|---|---|---|---|---|---|---|
| 1 | 2 | $10^5$ | i.v. | 10-12 | 128 | 7,5 | akute Hepatitis |
| 2 | 20 | $10^{7,8}$ | i.v. | 8-9 | 14 | 5,3 | portale Infiltrate, leichte, akute Hepatitis |
| 3 | 31 | $10^{7,4}$ | oral | 7-10 | 11 | negativ | unverändertes Lebergewebe |
| 4 | 51 | $10^{7,2}$ | oral | 5-10 | 11 | negativ | unverändertes Lebergewebe |
| 5 | 20 | $10^{7,8}$ | oral | 18-21 | 22 | 2,6 | unverändertes Lebergewebe |

B e l a s t u n g s i n f e k t i o n

| Schimpanse | Kulturpassage | HAV-Inokulum Dosis $(KID_{50})$ | Inokulation | GGTP (U/l) Normalwerte | max. Werte | Anti-HAV-Antikörper HAVAB (IU/ml) maximal | Histopathologie |
|---|---|---|---|---|---|---|---|
| 5 | 2 | $10^{5,0}$ | oral | s.oben | 27 | 7,5 | Einzellnekrosen, sonst normales Lebergewebe |

**Ansprüche**

1. Vaccine gegen Hepatitis A enthaltend eine wirksame Menge eines Hepatitis A-Virus, das aus menschlichem Material isoliert und ausschließlich in Zellen menschlichen Ursprungs vermehrt wurde, dadurch gekennzeichnet, daß zur Vermehrung des Virus fünfzehn-bis dreißigmal passagiert wurde.

2. Vaccine nach Anspruch 1, dadurch gekennzeichnet, daß zwanzig-bis dreißigmal passagiert wurde.

3. Vaccine nach Anspruch 1 in einer oral anwendbaren Zubereitung.

4. Vaccine nach Anspruch 2 in einer oral anwendbaren Zubereitung.

<u>Patentansprüche</u> <u>für</u> <u>folgenden</u> <u>Vertragsstaten</u> : <u>GR;</u> <u>ES</u>

1. Verfahren zur Herstellung einer Vaccine gegen Hepatitis A, dadurch gekennzeichnet, daß ein Hepatitis A-Virus aus menschlichem Material isoliert wird, daß ausschließlich in Zellen menschlichen Ursprungs vermehrt wird und daß eine wirksame Menge dieses Virus zu einer Vaccine aufgearbeitet wird, dadurch gekennzeichnet, daß zur Vermehrung des Virus fünfzehn-bis dreißigmal passagiert wird.

2. Verfahren nach Anspruch 1, dadurch gekennzeichnet, daß zwanzig-bis dreißigmal passagiert wird.

3. Verfahren nach Anspruch 1 oder 2, dadurch gekennzeichnet, daß zu einer oral anwendbaren Vaccine aufgearbeitet wird.